(19) Europäisches Patentamt
European Patent Office
Office européen des brevets

(11) Veröffentlichungsnummer: **0 017 711**
**B1**

(12) # EUROPÄISCHE PATENTSCHRIFT

(45) Veröffentlichungstag der Patentschrift: **07.04.82**

(51) Int. Cl.³: **C 07 C 43/225**, C 07 C 41/22, C 07 C 41/16

(21) Anmeldenummer: **80100595.0**

(22) Anmeldetag: **06.02.80**

(54) **Verfahren zur Herstellung von 2,2-Bis(4-(2,3-dibrompropoxy)-3,5-dibromphenyl)-propan.**

(30) Priorität: **13.02.79 DE 2905397**

(43) Veröffentlichungstag der Anmeldung: **29.10.80 (Patentblatt 80/22)**

(45) Bekanntmachung des Hinweises auf die Patenterteilung: **07.04.82 Patentblatt 82/14**

(84) Benannte Vertragsstaaten: **DE FR GB IT NL**

(56) Entgegenhaltungen: **DE - A - 2 226 694**

(73) Patentinhaber: **RIEDEL-DE HAEN AKTIENGESELLSCHAFT**
**Wunstorfer Strasse 40**
**D-3016 Seelze 1 (DE)**

(72) Erfinder: **Dannenberg, Wolfgang, Dr.**
**Leyserstrasse 7**
**D-3050 Wunstorf (DE)**
Erfinder: **Heyer, Walter, Dr.**
**Am Vogelbrink 20**
**D-3013 Barsinghausen (DE)**
Erfinder: **Döldissen, Theo, Dr.**
**Martinskirchstrasse 4a**
**D-3016 Seelze (DE)**
Erfinder: **Zimmermann, Manfred**
**Schneewittchenweg 22**
**D-3000 Hannover (DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 017 711 B1

Imprimerie Jouve, 18, rue St-Denis, 75001 Paris, France

Verfahren zur Herstellung von 2,2-Bis(4-(2,3-dibrompropoxy)-3,5-dibromphenyl)-propan

Die Erfindung betrifft 2,2-Bis[4-(2,3-dibrompropoxy)-3,5-dibromphenyl]-propan und Verfahren zu seiner Herstellung durch Umsetzung von 2,2-Bis[4-hydroxy-3,5-dibromphenyl]-propan mit Allylchlorid und Bromierung des so erhaltenen Diallyläthers mit Brom.

2,2-Bis[4-(2,3-dibrompropoxy)-3,5-dibromphenyl]-propan (im folgenden BDDP genannt) ist ein Derivat des Tetrabrombisphenol A und aus der deutschen Offenlegungsschrift 22 10 916 (= US-Patentschrift 3 904 694) bekannt. Es wird als Flammschutzmittel, häufig in Verbindung mit Antimontrioxid, in der Kunststoffindustrie verwendet. Es hat die folgende Formel :

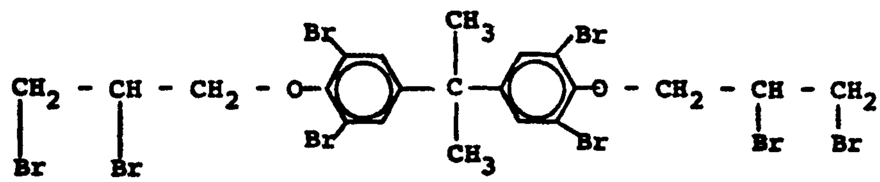

Verschiedene Verfahren zur Herstellung von BDDP sind bekannt :

So wird gemäß deutscher Offenlegungsschrift 22 26 694 (= britischer Patentschrift 13 34 638) und deutscher Auslegeschrift 25 52 713 Tetrabrombisphenol A (2,2-Bis(4-hydroxy-3,5-dibromphenyl)-propan) (im folgenden BHDP genannt) in alkalisch alkoholischer Lösung veräthert und das Reaktionsprodukt, der BHDP-Diallyläther, isoliert. Anschließend wird die so erhaltene Verbindung in Chloroform oder Tetrachlorkohlenstoff bromiert, und das Lösungsmittel wird durch Destillation entfernt. Das anfallende Produkt ist eine hochviskose gelbliche Flüssigkeit, die nach längerem Stehen durchhärtet. In der deutschen Offenlegungsschrift 22 10 916 erfolgt die Bromierung und anschließende Fällung des ebenfalls isolierten BHDP-Diallyläthers in einer Monocarbonsäure, z.B. Essigsäure. Das Produkt fällt in suspendierter Form an, das nach bekannten Verfahren abgetrennt und aufbereitet wird. Nach der japanischen Patentveröffentlichung Nr. 74 125 348 wird zunächst in Methylenchlorid bromiert, und dann wird das Produkt nach Abdestillieren des Lösungsmittels zur Erzielung eines höheren Schmelzbereiches mit einem aliphatischen Keton zur Kristallisation aufgenommen.

Nach den bekannten Verfahren ist es in jedem Fall notwendig, den BHDP-diallyläther als kristallines Zwischenprodukt zu isolieren, um die nachfolgende Bromierung ohne Qualitätseinbußen durchführen zu können. Bei Abtrennungsprozessen dieser Art muß immer mit höherem Arbeitsaufwand und Ausbeuteverlusten gerechnet werden. Es werden sehr unterschiedliche Produktqualitäten erhalten, zum Teil hochviskose oder erstarrte Flüssigkeiten mit niedrigem Schmelzbereich oder feinkristalline bis puderartige Pulver, die bei Weiterverarbeitung im Mischvorgang zu Verklebungen neigen können. Darüberhinaus macht die Verwendung von Chloroform als Lösungsmittel wegen der dem Fachmann bekannten Giftigkeit erhöhte Sicherheitsvorkehrungen notwendig. Essigsäure ist aufgrund ihrer Löslichkeitseigenschaften und der schwierigen Abtrocknung kein ideales Bromierungsmittel. Die Verwendung von aliphatischen Ketonen als Kristallisationsmittel führt zwar zu höherschmelzenden Qualitäten, hat jedoch auch eine höhere Löslichkeit des Endproduktes und damit Ausbeuteminderung zur Folge.

Es bestand daher die Aufgabe, ein Verfahren zur Herstellung von BDDP zu entwickeln, das den Herstellprozeß vereinfacht, zu einer Ausbeuteerhöhung führt und eine Produktform liefert, die ohne Schwierigkeiten weiterverarbeitet werden kann.

Die Erfindung betrifft nun 2,2-Bis[4-(2,3-dibrompropoxy)-3,5-dibromphenyl]-propan, hergestellt durch (a) Umsetzung von 2,2-Bis[4-hydroxy-3,5-dibromphenyl]-propan mit einem Allylhalogenid in Lösung in einem Gemisch aus Wasser und einem niederen aliphatischen Alkohol, (b) Entfernung des niederen aliphatischen Alkohols, (c) Auflösung des nach (b) erhaltenen Allyläthers in einem aromatischen Halogenkohlenwasserstoff und Entfernung des Wassers, (d) Bromierung des Allyläthers mit Brom, (e) Entfernung des überschüssigen Broms, (f) Waschen der nach (e) erhaltenen Bromallyläther-Lösung mit Wasser, (g) Fällung des Bromallyläthers mittels eines niederen aliphatischen Alkohols und anschließende Abtrennung.

Die Erfindung betrifft ferner ein Verfahren zur Herstellung von 2,2-Bis[4-(2,3-dibrompropoxy)-3,5-dibromphenyl]-propan durch Umsetzung von 2,2-Bis[4-hydroxy-3,5-dibromphenyl]-propan mit einem Allylhalogenid in alkoholischer Lösung und Bromierung des erhaltenen Allyläthers mit Brom, dadurch gekennzeichnet, daß (a) 2,2-Bis[4-hydroxy-3,5-dibromphenyl]-propan mit dem Allylhalogenid in Lösung in einem Gemisch aus Wasser und einem niederen aliphatischen Alkohol umgesetzt wird, (b) der niedere aliphatische Alkohol entfernt wird, (c) der nach (b) erhaltene Allyläther in einem aromatischen Halogenkohlenwasserstoff gelöst und das Wasser entfernt wird, (d) der Allyläther mit Brom bromiert wird, (e) das überschüssige Brom entfernt wird, (f) die nach (e) erhaltene Bromallyläther-Lösung mit Wasser gewaschen wird, (g) der Bromallyläther mittels eines niederen aliphatischen Alkohols gefällt und dann abgetrennt wird.

Das Ausgangsmaterial BHDP ist nach bekannten Verfahren herstellbar, z.B. durch Bromieren von 2,2-Bis[4-hydroxyphenyl]-propan (= Bisphenol A) mit 4 bis 4,5 Mol elementarem Brom pro Mol Bisphenol in

einem aliphatischen, vorzugsweise wäßrigen, Alkohol mit 1 bis 4 Kohlenstoffatomen bei einer Temperatur von 15 bis 45 °C (vgl. deutsche Patentschrift 1 268 149 = US-Patentschrift 3 029 291). Dabei wird vorzugsweise ein Gemisch aus Methanol und Wasser im Gewichtsverhältnis von 1 : 1 bis 3 : 1 verwendet. Das Reaktionsgemisch kann zur Vervollständigung der Reaktion abschließend noch eine Zeitlang auf die Siedetemperatur des Alkohol/Wasser-Gemisches erhitzt werden.

Das BHDP wird in wäßrig alkoholischer Lösung mit einem Allylhalogenid, z.B. Allylbromid und vorzugsweise Allylchlorid, zum entsprechenden Allyläther umgesetzt. Als Alkohol wird ein niederer aliphatischer Alkohol mit vorzugsweise 1, 2 oder 3 Kohlenstoffatomen eingesetzt, z.B. Methanol, Äthanol, n-Propanol und Isopropanol ; die Verwendung von Methanol ist bevorzugt. Das Gewichtsverhältnis von Wasser zu Alkohol beträgt hierbei 30 : 70 bis 70 : 30, vorzugsweise 45 : 55 bis 55 : 45. Die Allylierung wird bei einer Temperatur von 35 bis 45 °C, vorzugsweise 38 bis 42 °C, in alkalischem Medium durchgeführt. Der pH-Wert des Mediums liegt im Bereich von 11,5 bis 13,0, vorzugsweise 11,8 bis 12,2 ; er wird mit Hilfe einer anorganischen Base erreicht, vorzugsweise mit einem Alkalihydroxid wie insbesondere Natriumhydroxid oder Kaliumhydroxid. Die Beendigung der Umsetzung zeigt sich durch das Auftreten einer starken Fällung und Änderung des pH-Wertes gegen 7.

Anschließend wird der Alkohol aus dem Reaktionsgemisch entfernt, vorzugsweise durch Destillation. Diese wird gegebenenfalls bei vermindertem Druck durchgeführt. Die erhaltene wäßrige Suspension wird dann mit einem aromatischen Halogenkohlenwasserstoff versetzt, und zwar in einer Menge, die zur Auflösung des ausgefällten Allyläthers ausreicht. Insbesondere wird der Halogenkohlenwasserstoff in einer Menge von 0,5 bis 2 l, vorzugsweise von 1,2 bis 1,6 l (bezogen auf 1 Mol BHDP) eingesetzt. Der Allyläther wird in dem Halogenkohlenwasserstoff gelöst, und es bilden sich zwei gut trennbare Phasen.

Als aromatische Halogenkohlenwasserstoffe eignen sich vor allem einfach, zweifach oder dreifach halogenierte aromatische Kohlenwasserstoffe, die vorzugsweise 6 oder 7 Kohlenstoffatome aufweisen. Beispiele hierfür sind Chlorbenzol, Brombenzol, Fluorbenzol, 2-Chlortoluol, 2-Bromtoluol, 2-Fluortoluol, 1,2-Dichlorbenzol und Benzotrichlorid. Statt eines einzelnen Halogenkohlenwasserstoffs kann auch ein Gemisch verschiedener Halogenkohlenwasserstoffe eingesetzt werden.

Die Lösung des Allyläthers in dem aromatischen Halogenkohlenwasserstoff wird ohne jede weitere Aufarbeitung zur Bromierung verwendet ; eine Isolierung des Allyläthers ist nicht notwendig. Überraschenderweise wird durch diese Vereinfachung des Verfahrens weder die Produktqualität noch die Ausbeute beeinträchtigt.

Die Bromierung des Allyläthers in dem aromatischen Halogenkohlenwasserstoff wird mit elementarem Brom durchgeführt. Dieses wird in einer Menge von 2,0 bis 2,4 Mol, vorzugsweise 2,2 bis 2,3 Mol, pro Mol Allyläther eingesetzt. Die Bromierung erfolgt üblicherweise bei einer Temperatur von 10 bis 30 °C, vorzugsweise 15 bis 25 °C.

Nach der Bromierung wird überschüssiges Brom entfernt. Dies erfolgt vorzugsweise mit Hilfe von Natriumbisulfit. Anschließend wird die erhaltene Bromallyläther (BDDP)-Lösung mit Wasser gewaschen. Mehrmaliges Waschen bei einer Temperatur von 30 bis 50 °C, vorzugsweise 35 bis 45 °C, ist dabei vorteilhaft.

Aus der durch Waschen gereinigten Lösung wird das 2,2-Bis-[4-(2,3-dibrompropoxy)-3,5-dibromphenyl]-propan durch Vermischen mit einem niederen aliphatischen Alkohol gefällt. Hierfür kommen die oben erwähnten Alkohole in Betracht, vorzugsweise Methanol ; sie werden üblicherweise im Überschuß verwendet. Die Fällung wird bei einer Temperatur von 10 bis 30 °C, vorzugsweise von 15 bis 25 °C, durchgeführt. Der Zusatz von Impfkristallen ist vorteilhaft ; ferner empfiehlt es sich, das Gemisch in ständiger Bewegung zu halten, vorzugsweise durch Rühren. Besonders vorteilhaft ist die Fällung des BDDP durch Einleiten der BDDP-Lösung in dem aromatischen Kohlenwasserstoff in das ein- bis vierfache Volumen des niederen aliphatischen Alkohols. Das ausgefällte BDDP wird schließlich abgetrennt, vorzugsweise durch Absaugen der flüssigen Bestandteile des Fällungsgemisches, und bei einer Temperatur von 35 bis 70 °C getrocknet.

Das erfindungsgemäße BDDP fällt in Form eines reinweißen Granulats an. Das Granulat weist durchschnittliche Partikeldurchmesser von vorzugsweise 500 bis 1 000 µm auf. Der Schmelzbereich liegt bei 95 bis 110 °C, und das mittlere Schüttgewicht beträgt 0,7 bis 1,0 g/$cm^3$. Das Granulat neigt nicht zum Verkleben oder zur Belagsbildung bei der Weiterverarbeitung. Es zeigt ferner eine ausgezeichnete Rieselfähigkeit.

Die folgenden Beispiele dienen der Erläuterung der Erfindung. Prozentangaben beziehen sich auf das Gewicht.

## Beispiel 1

In einem 5 $m^3$-Behälter aus gummiertem Stahl mit Destillieraufsatz werden
1 000 l Trinkwasser vorgelegt und darin unter Rühren
179 kg Natriumhydroxid (4,48 kmol) gelöst. Dazu werden
1 260 l Methanol und
1 100 kg BHDP (Tetrabrombisphenol A) (2 kmol) nacheinander hinzugefügt. Es entsteht eine hellbraune klare Lösung mit einem pH Wert von ca. 12.

Die klare Lösung wird nach Anheizen auf 40 °C schubweise während einer Stunde mit

376 kg Allylchlorid (4,88 kmol), versetzt. Dabei wird die Temperatur bei 40 °C gehalten. Nach erfolgter Zugabe des Allylchlorids wird die Reaktionsmischung 5 Stunden lang im Sieden gehalten. Die Sumpftemperatur steigt dabei von ca. 44 °C auf ca. 47 °C. Es tritt eine starke Fällung auf. Der pH-Wert liegt gegen Ende der Reaktion bei ca. 7.

Aus dem Reaktionsgemisch werden etwa

1 000 l Methanol abdestilliert. Nach Abkuhlung auf ca. 60 °C Sumpftemperatur werden auf den Rückstand unter ständigem Rühren

1 500 l (1 650 kg) Chlorbenzol gegeben, was zur Auflösung der Fällung und zur Ausbildung von zwei gut separierbaren Phasen führt. Von dem Gemisch wird die wässrige obere Phase gründlich abgetrennt.

Die Chlorbenzolphase wird anschließend bromiert : In die klare Lösung werden bei Temperaturen um 20 °C unter Rühren und leichter Kühlung

728 kg Brom (4,55 kmol) eindosiert. Anschließend läßt man eine Std. nachrühren. Zur Reduktion der überschüssigen Brommengen werden

1 500 l Wasser unter Zusatz von

50 kg Natriumbisulfit zugesetzt und gerührt. Ist das Reaktionsgemisch hellgelb, wird noch 3 mal mit je

500 l gewaschen. Mit Soda wird auf einen pH-Wert von 6 bis 6,5 gestellt und gründlich das letzte Wasser abgeschöpft. Die Temperatur des Reaktionsgemisches wird während des gesamten Waschvorgangs auf 40 °C gehalten. Zur Fällung des Ansatzes werden in einem anderen Behälter aus gummiertem Stahl von 10 $m^3$ Fassungsvermögen

4 800 l Methanol (= 3 800 kg) vorgelegt und mit 25 kg Impfkristallen versetzt. Bei Temperaturen um 20 °C läßt man die Chlorbenzollösung zunächst langsam, dann gegen Ende der Fällung schnell einlaufen. Es fällt ein körniges, rein weißes Granulat aus. Dieses wird sofort nach Beendigung des Fällvorganges über eine Nutsche abgesaugt, mit Methanol gewaschen und getrocknet.

Die Mutterlauge kann fraktioniert destilliert und die erhaltenen Methanol- und Chlorbenzolfraktionen können direkt wieder verwendet werden.

Nach dem Trocknen der abgesaugten Ware bei 60 °C im Umlufttrockenschrank beträgt die Ausbeute

1 800 kg BDDP (94,3 % der Theorie bezogen auf BHDP).

Das Produkt ist ein körniges weißes Granulat, mit einer durchschnittlichen Teilchengröße von ca. 500 µm, einem Bromgehalt von 65-67 %, einem Schmelzbereich von 100-110 °C und einem Schüttgewicht von ca. 0,7 g/$cm^3$. Bei Erhitzung der Substanz auf eine Temperatur von über 200 °C tritt eine leichte Gelbfärbung der Schmelze auf.

## Beispiel 2

In ein Lösungsmittelgemisch aus

250 ml Trinkwasser und

315 ml Methanol werden unter Rühren

44,7 g Natriumhydroxid (1,12 Mol) und

275 g BHDP (Tetrabrombisphenol A) (0,5 Mol) nacheinander eingetragen. Nach einiger Zeit entsteht eine hellbraune klare Lösung mit einem pH-Wert von ca. 12.

Die Lösung wird nach Aufheizen auf 40 °C tropfenweise mit

94 g Allylchlorid (1,22 Mol) versetzt, wobei die Temperatur auf 40 °C gehalten wird. Nach erfolgter Zugabe des Allylchlorids wird die Reaktionsmischung 5 Stunden unter Rückfluß erhitzt. Es tritt eine starke Fällung auf ; der pH-Wert liegt gegen Ende der Reaktion bei ca. 7.

Aus dem Reaktionsgemisch werden etwa

250 ml Methanol abdestilliert. Nach Abkühlung auf ca. 60 °C Sumpftemperatur wird der Rückstand unter ständigem Rühren mit

375 ml Brombenzol (= 562 g), versetzt, was zur Auflösung der Fällung und zur Ausbildung von zwei gut separierbaren Phasen führt. Von dem Gemisch wird die wäßrige obere Phase abgetrennt und verworfen. Die Brombenzolphase wird anschließend unter Rühren und leichter Außenkühlung bei Temperaturen um 20 °C bromiert.

Dazu werden

182 g Brom (1,14 Mol) tropfenweise zugesetzt und eine Stunde nachgerührt. Zur Reduktion der überschüssigen Brommenge wird

250 ml Trinkwasser unter Zusatz von

12 g Natriumbisulfit zugesetzt und gerührt.

Wenn die Bromfarbe verschwunden ist, wird nach Abtrennung der wäßrigen Phase noch 3 mal mit je

200 ml Wasser gewaschen (bei 40 °C) und das letzte Waschwasser gründlich abgetrennt.

Zur Fällung werden in einem anderen Gefäß

1 200 ml Methanol (= 950 g), mit

7,5 g Impfkristallen vorgelegt. Bei Temperaturen um 20 °C läßt man die Brombenzollösung zunächst langsam, dann gegen Ende der Fällung schnell einlaufen. Es fällt ein körniges, rein weißes Granulat aus, das nach Beendigung der Fällung über eine Nutsche abgesaugt, mit

200 ml Methanol gewaschen und bei 60 °C getrocknet wird. Die Ausbeute beträgt nach der Trocknung

407 g BDDP (86 % d. Theorie, bezogen auf BHDP). Die durchschnittliche Teilchengröße liegt bei 1 000 µm, der Bromgehalt zwischen 65-67 %. Der Schmelzbereich liegt bei 95,5-100 °C, das Schüttgewicht beträgt ca. 0,7 g/cm³.

Beispiel 3

Beispiel 2 wird wiederholt unter Verwendung von 500 ml Fluorbenzol. Es wird die gleiche Ausbeute erreicht. Das erhaltene Produkt entspricht dem in Beispiel 2 erhaltenen, weist jedoch eine durchschnittliche Korngröße von 500-800 µm auf.

In analoger Weise läßt sich das vorstehend genannte Verfahren mit Benzotrichlorid, Chlortoluol, Fluortoluol, Dichlorbenzol als halogenierten Aromaten durchführen.

**Ansprüche**

1. 2,2-Bis[4-(2,3-dibrompropoxy)-3,5-dibromphenyl]-propan, hergestellt durch (a) Umsetzung von 2,2-Bis[4-hydroxy-3,5-dibromphenyl]-propan mit einem Allylhalogenid in Lösung in einem Gemisch aus Wasser und einem niederen aliphatischen Alkohol, (b) Entfernung des niederen aliphatischen Alkohols, (c) Auflösung des nach (b) erhaltenen Allyläthers in einem aromatischen Halogenkohlenwasserstoff und Entfernung des Wassers, (d) Bromierung des Allyläthers mit Brom, (e) Entfernung des überschüssigen Broms, (f) Waschen der nach (e) erhaltenen Bromallyläther-Lösung mit Wasser, (g) Fällung des Bromallyläthers mittels eines niederen aliphatischen Alkohols und anschließende Abtrennung.

2. 2,2-Bis[4-(2,3-dibrompropoxy)-3,5-dibromphenyl]-propan nach Anspruch 1, dadurch gekennzeichnet, daß es als Granulat mit einem durchschnittlichen Partikeldurchmesser von 500 bis 1 000 µm vorliegt.

3. 2,2-Bis[4-(2,3-dibrompropoxy)-3,5-dibromphenyl]-propan nach Anspruch 1, dadurch gekennzeichnet, daß es ein mittleres Schüttgewicht von 0,7 bis 1,0 g/cm³ aufweist.

4. Verfahren zur Herstellung von 2,2-Bis[-4-(2,3-dibrompropoxy)-3,5-dibromphenyl]-propan durch Umsetzung von 2,2-Bis[4-hydroxy-3,5-dibromphenyl]-propan mit einem Allylhalogenid in alkoholischer Lösung und Bromierung des erhaltenen Allyläthers mit Brom, dadurch gekennzeichnet, daß (a) 2,2-Bis[4-hydroxy-3,5-dibromphenyl]-propan mit dem Allylhalogenid in Lösung in einem Gemisch aus Wasser und einem niederen aliphatischen Alkohol umgesetzt wird, (b) der niedere aliphatische Alkohol entfernt wird, (c) der nach (b) erhaltene Allyläther in einem aromatischen Halogenkohlenwasserstoff gelöst und das Wasser entfernt wird, (d) der Allyläther mit Brom bromiert wird, (e) das überschüssige Brom entfernt wird, (f) die nach (e) erhaltene Bromallyläther-Lösung mit Wasser gewaschen wird, (g) der Bromallyläther mittels eines niederen aliphatischen Alkohols gefällt und dann abgetrennt wird.

5. Verfahren nach Anspruch 4, dadurch gekennzeichnet, daß als niederer aliphatischer Alkohol ein Alkanol mit 1, 2 oder 3 Kohlenstoffatomen verwendet wird.

6. Verfahren nach Anspruch 4, dadurch gekennzeichnet, daß als aromatischer Halogenkohlenwasserstoff ein einfach, zweifach oder dreifach halogenierter aromatischer Kohlenwasserstoff mit 6 oder 7 Kohlenstoffatomen verwendet wird.

7. Verfahren nach Anspruch 4, dadurch gekennzeichnet, daß der aromatische Halogenkohlenwasserstoff in einer Menge von 0,5 bis 2 l pro Mol Allyläther eingesetzt wird.

8. Verfahren nach Anspruch 4, dadurch gekennzeichnet, daß die Fällung des Bromallyläthers durch Einleiten der Lösung des Bromallyläthers in dem aromatischen Halogenkohlenwasserstoff in das ein- bis vierfache Volumen des niederen Alkohols durchgeführt wird.

**Claims**

1. 2,2-Bis[4-(2,3-dibromopropoxy)-3,5-dibromophenyl]-propane, obtained by (a) reaction of 2,2-bis[4-hydroxy-3,5-dibromophenyl]-propane with an allyl halide in solution in a mixture of water and a lower aliphatic alcohol, (b) removal of the lower aliphatic alcohol, (c) dissolution of the allyl ether obtained according to (b) in an aromatic halogenated hydrocarbon and removal of the water, (d) bromination of the allyl ether with bromine, (e) removal of excess bromine, (f) washing of the bromoallyl ether solution obtained according to (e) with water, (g) precipitation of the bromoallyl ether by means of a lower aliphatic alcohol, and subsequent separation.

2. 2,2-Bis[4-(2,3-dibromopropoxy)-3,5-dibromophenyl]-propane according to Claim 1, characterised in that it is present in the form of granules having an average particle diameter of 500 to 1,000 µm.

3. 2,2-Bis[4-(2,3-dibromopropoxy)-3,5-dibromophenyl]-propane according to Claim 1, characterised in that it has an average apparent density of 0.7 to 1.0 g/cm³.

4. Process for the manufacture of 2,2-bis[4-(2,3-dibromopropoxy)-3,5-dibromophenyl]-propane by reaction of 2,2-bis[4-hydroxy-3,5-dibromophenyl]-propane with an allyl halide in an alcoholic solution and

bromination of the allyl ether obtained with bromine, characterised in that (a) 2,2-bis[4-hydroxy-3,5-dibromophenyl]-propane is reacted with the allyl halide in solution in a mixture of water and a lower aliphatic alcohol, (b) the lower aliphatic alcohol is removed, (c) the allyl ether obtained according to (b) is dissolved in an aromatic halogenated hydrocarbon and the water is removed, (d) the allyl ether is brominated with bromine, (e) the excess bromine is removed, (f) the bromoallyl ether solution obtained according to (e) is washed with water, (g) the bromoallyl ether is precipitated by means of a lower aliphatic alcohol, and subsequently separated.

5. Process according to Claim 4, characterised in that as lower aliphatic alcohol an alkanol having 1, 2 or 3 carbon atoms is used.

6. Process according to Claim 4, characterised in that as aromatic halogenated hydrocarbon a mono-, di- or trihalogenated aromatic hydrocarbon having 6 or 7 carbon atoms is used.

7. Process according to Claim 4, characterised in that the aromatic halogenated hydrocarbon is used in an amount of from 0.5 to 2 liters per mol of allyl ether.

8. Process according to Claim 4, characterised in that the precipitation of the bromoallyl ether is performed by introducing the solution of the bromoallyl ether in the aromatic halogenated hydrocarbon into the 1- to 4-fold volume of the lower alcohol.

**Revendications**

1. Bis-[(dibromo-2,3 propoxy)-4 dibromo-3,5 phényl]-2,2 propane qui a été préparé par les opérations suivantes : (a) réaction du bis-(hydroxy-4 dibromo-3,5 phényl)-2,2 propane avec un halogénure d'allyle en solution dans un mélange d'eau et d'un alcool aliphatique inférieur, (b) élimination de l'alcool aliphatique inférieur, (c) dissolution de l'éther allylique obtenu sous (b) dans un hydrocarbure aromatique halogéné et élimination de l'eau, (d) bromation de l'éther allylique par du brome, (e) élimination de l'excès de brome, (f) lavage à l'eau de la solution d'éther bromallylique obtenue sous (e), (g) précipitation de l'éther bromallylique au moyen d'un alcool aliphatique inférieur, puis séparation.

2. Bis-[(dibromo-2,3 propoxy)-4 dibromo-3,5 phényl]-2,2 propane selon la revendication 1, caractérisé en ce qu'il est sous la forme d'un granulé dont le diamètre moyen des particules est compris entre 500 et 1 000 μm.

3. Bis-[(dibromo-2,3 propoxy)-4 dibromo-3,5 phényl]-2,2 propane selon la revendication 1, caractérisé en ce qu'il a une masse volumique apparente moyenne de 0,7 à 1,0 g/cm$^3$.

4. Procédé de préparation du bis-[(dibromo-2,3 propoxy)-4 dibromo-3,5 phényl]-2,2 propane par réaction du bis-(hydroxy-4 dibromo-3,5 phényl)-2,2 propane avec un halogénure d'allyle en solution alcoolique et bromation de l'éther allylique obtenu au moyen du brome, procédé caractérisé en ce que (a) on fait réagir le bis-(hydroxy-4 dibromo-3,5 phényl)-2,2 propane avec l'halogénure d'allyle en solution dans un mélange d'eau et d'un alcool aliphatique inférieur, (b) on élimine l'alcool aliphatique inférieur, (c) on dissout l'éther allylique obtenu sous (b) dans un hydrocarbure aromatique halogéné et on chasse l'eau, (d) on brome l'éther allylique par du brome, (e) on chasse l'excès de brome, (f) on lave à l'eau la solution d'éther bromallylique obtenue sous (e), (g) on fait précipiter l'éther bromallylique au moyen d'un alcool aliphatique inférieur, puis on le sépare.

5. Procédé selon la revendication 4, caractérisé en ce qu'on utilise, comme alcool aliphatique inférieur, un alcanol contenant 1, 2 ou 3 atomes de carbone.

6. Procédé selon la revendication 4, caractérisé en ce qu'on utilise, comme hydrocarbure aromatique halogéné, un hydrocarbure aromatique monohalogéné, dihalogéné ou trihalogéné qui contient 6 ou 7 atomes de carbone.

7. Procédé selon la revendication 4, caractérisé en ce que l'hydrocarbure aromatique halogéné est mis en jeu en une quantité de 0,5 à 2 l par mole de l'éther allylique.

8. Procédé selon la revendication 4, caractérisé en ce qu'on introduit la solution de l'éther bromallylique dans l'hydrocarbure aromatique halogéné, pour faire précipiter l'éther bromallylique, dans un à quatre fois son volume de l'alcool inférieur.